# EUROPEAN PATENT SPECIFICATION

(11) **EP 2 467 133 B1**
(45) Date of publication and mention of the grant of the patent: **22.01.2014**
(21) Application number: 10742380.8
(22) Date of filing: 03.08.2010
(51) Int. Cl.: A61K 9/70, A61L 15/44, A61L 15/58, A61K 8/89, A61Q 19/00

(54) **MULTI-LAYER TRANSDERMAL PATCH**
MEHRSCHICHTIGES TRANSDERMALES PFLASTER
TIMBRE TRANSDERMIQUE MULTICOUCHE

(30) Priority: 18.08.2009 EP 09305763
(43) Date of publication of application: 27.06.2012
(73) Proprietor: Dow Corning Corporation, Midland, Michigan 48686-0994 (US); Dow Corning France SAS, 69432 Lyon Cedex 03 (FR)
(72) Inventor: COLAS, Andre, B-1330 Rixensart (BE); SCHALAU, Gerald, K., Freeland MI 48623 (US); THOMAS, Xavier, F-59300 Famars (FR)
(74) Representative: Thomson, Craig Richard
(86) International application number: PCT/US2010/044184
(87) International publication number: WO 2011/022199

(56) References cited:
- EP-A2- 1 025 843
- WO-A2-2007/113597
- WO-A2-2010/121033
- FR-A1- 2 735 024
- US-A1- 2005 282 977

## Description

### BACKGROUND OF THE INVENTION

Silicone gel adhesives (SGA) are two-part adhesives that when mixed and fully cured possess the tack properties associated with pressure sensitive adhesives and the resiliency of a soft elastomeric matrix. It has been hypothesized that a therapeutic patch to deliver an active that employs a SGA as the skin contacting adhesive may be beneficial, because, as a class, the SGAs exhibit no cold flow or sensitivity to plasticizing effects. These properties, coupled with their ease of removal from skin, have enabled the SGAs to become the adhesive of choice in many advanced wound care and scar care applications. It is known that scar dressings prepared with SGA as the skin interface can be removed from the skin and reapplied. This may also prove a benefit in certain therapies including OTC patches for acute muscle soreness where a patch could be used on multiple areas or for numerous times until it no longer was providing relief.

Many of the therapeutic patches on the market today are of the "matrix type", where the active pharmaceutical as well as any necessary permeation enhancers or release modulators are blended directly into the adhesive. The adhesive/drug mixture is then devolatized or cured as necessary to create a laminate that can be cut to the appropriate dimensions. Matrix patches using SGA as the adhesive have been prepared, but the actives that can be successfully incorporated into the matrix are limited. It has been found that many drugs including lidocaine, niacinamide, econazole nitrate, ketoprofen, nicotine and salicylic acid either completely poisoned the cure or required special handling and curing procedures in producing the laminate to achieve a cohesive laminate.

This invention overcomes the problems with using a SGA and a pressure sensitive adhesive (PSA). The drug is incorporated into a pressure sensitive adhesive (PSA) layer that is then covered with a SGA wherein the SGA is the skin contacting adhesive. Lidocaine, salicylic acid and econazole nitrate particularly benefit by the dual adhesive construction of this invention.

Lidocaine is indicated as a local anesthetic. At least one lidocaine patch is already commercially available (e.g. Lidoderm®) and can provide a steady source of the drug over time. The current product uses a hydrogel adhesive to achieve a low trauma removal from the skin. Many topical formulations also exist. A patch should alleviate discomfort for a longer time than a traditionally prepared topical formulation that may be wiped or sloughed off. A dual adhesive patch would also use to its advantage many of the aforementioned SGA characteristics, including conformability, a reasonable adhesion and low peel force from skin. Wound healing properties of dressings or topical patches prepared with SGA and lidocaine may also benefit from the adhesive's characteristic occlusivity. The occlusivity may also improve release rates of the drug into the skin.

Salicylic acid is currently used as a topical wart and corn remover at high concentrations and an anti-acne treatment at lower concentrations. Most formulations utilize either the slight antiseptic action or the considerable keratolytic action salicylic acid imparts. Most treatments in which salicylic acid is indicated involve compromised or broken skin, so the low peel force from skin associated with SGA could be a benefit.

Econazole nitrate is a lipophilic drug that is indicated as an antifungal. It is currently used in topical antifungal applications, and is being investigated for transdermal antifungal uses. Previous work has demonstrated that econazole nitrate will negatively impact the cure of SGAs.

WO2007/113597 discloses a realisably adhesive laminate comprising a structural layer carrying on at least part of one side thereof a hydrophobic gel and on at least part of the other side thereof a pressure-sensitive adhesive. The structural layer is preferably a film of synthetic plastics material, e.g. polyurethane. The pressure-sensitive adhesive is preferably an acrylic adhesive, and the hydrophobic gel is preferably a silicone gel.

FR2735024 discloses adhesive patches for cutaneous administration of a medicine or cosmetic comprise a silicone adhesive gel and a polymeric support, with the active material incorporated in the silicone gel.

US2005/282977 discloses a pressure sensitive adhesive composition comprising a compatible blend of a gel preferably comprising a copolymer of an organosiloxane and a siloxane having a plurality of reactive functionalities reacted with a cross-liking agent and a pressure sensitive adhesive, wherein the pressure sensitive adhesive is a silicone pressure sensitive adhesive and has a higher adhesive strength than said gel and is blended with said copolymer during cross-linking of said copolymer to form said gel.

WO2010/121033 refers to a silicone gel adhesive construction comprising a porous backing and an acrylic copolymer pressure sensitive adhesive layer on which a cured silicone gel adhesive is applied.

### BRIEF SUMMARY OF THE INVENTION

This invention pertains to a construction consisting of in the order from the outside towards the inside: An occlusive or non occlusive external film layer; a non-curing pressure sensitive adhesive (PSA) that has been blended with a therapeutic concentration of at least one or a combination of cosmetic or pharmaceutical active ingredients; and a silicone gel adhesive that is used as the skin contact layer, as defined in the claims.

### DETAILED DESCRPTION OF THE INVENTION

Described herein is a construction comprising an external film layer; a non-curing pressure sensitive adhesive (PSA) that has been blended with a therapeutic concentration of at least one or a combination of cosmetic or pharmaceutical active ingredient; and a silicone cured elastomeric tacky gel or soft skin adhesive (SSA). Typically the constructions are used as transdermal patches to deliver the cosmetic or pharmaceutical active ingredient to a human.

As used herein, a "silicone gel" is an elastic, jelly-like solid material formed by lightly cross-linking silicone polymers. By contrast, a "PSA" has low or no resiliency.

### External Film Layer

The first element in the construction is an external film layer. The primary purpose of the external film layer is to form a layer over the pressure sensitive adhesive to prevent the construction from adhering via the pressure sensitive adhesive to clothing or other objects that the construction may come into contact with. The external film layer also contributes to the wear strength, tensile strength and tear strength of the construction. Film layers produced from silicone, fabric, coated papers, aluminum, plastics including polyesters, polyurethanes, high or low density polyethylene, polyvinyl acetate, polyvinylidene chloride ethyl vinyl acetate, polyethylene terephatlate, polybutylene terephthalate, polycarbonate, polyurethanes, or the like or any combinations thereof are useful herein. Typically the external film layer will have a thickness of less than 0.15 mm, alternatively 0.012 mm to 0.1 mm, alternatively 0.0625 mm to 0.0875 mm.

The external film layer may be occlusive or non-occlusive. By occlusive it is meant that the external film layer is partially or essentially impenetrable to microorganisms and/or other substances that may be detrimental the underlying skin or cause the cosmetic or pharmaceutical active ingredient to loose its effectiveness. Additionally, an occlusive film may be one that prevents transepidermal water loss which is believed to increase the permeability capability of the skin.

### Pressure Sensitive Adhesive

A non-curing pressure sensitive adhesive (PSA) is adhered to one side of the external film. The PSA can include any viscoelastic material which adheres instantaneously to most substrates with application of slight pressure and essentially remains tacky through the useful life of the construction. The non-curing PSA is silicone.

By non-curing it is meant that the adhesive are thermoplastic and are applied by hot-melt or a solvent based process and typically do not undergo further curing to solidify.

Silicone PSAs may be produced by methods known in the art, however, silicone PSAs produced from the catalyzed reaction of a SiH and C=C compound are not useful herein.

Silicone PSAs useful herein generally comprise the product of (I) a silanol-terminated polydiorganosiloxane crosslinked with (II) a silanol-containing silicone resin. In the organic substituents of the silanol-terminated polydiorganosiloxane are generally alkyl groups having 1 to 6 carbon atoms or phenyl groups. Typically at least 80% of the organic substituents are methyl groups. Alternatively, the silanol terminated polydiorganosiloxane is a dimethylhydroxy-terminated polydimethylsiloxane. The silanol-terminated polydiorganosiloxane typically has a viscosity of 0.1 Pa.s to 30000 Pa.s, alternatively 1 Pa.s to 100 Pa.s at 25°C. Methods for producing silanol-terminated polydiorganosiloxane are well known in the art.

The silanol-containing silicone resin (II) is typically a non-linear siloxane resin and consists of siloxane units of the formula R¹ₐSiO_{(4-a)/2} where R¹ is a hydroxyl, hydrocarbon or hydrocarbonoxy group and a has an average value of from 1 to 1.8. Typically the resin is comprised of groups having the formula R²₃SiO_{1/2} ("M" groups) and groups having the formula SiO_{4/2} ("Q" groups) where R² is a alkyl group having 1 carbon to 6 carbon atoms, typically methyl. The number ratio of M groups to Q groups is typically in the range of 0.05:1 to 1.2:1 (equivalent to the value of a in the formula R¹ₐSiO_{(4-a)/2} of 1.0 to 1.63), alternatively 0.6:1 to 0.9:1. The silicone resin typically contains at least 0.2 wt% to 5 wt%, alternatively 0.5 wt% to 3 wt% silicon-bonded hydroxy groups. These silicon-bonded hydroxy groups are typically present as M groups: (OH)(CH₃)₂SiO_{1/2}.

The silicone PSA typically is produced from 20 parts to 80 parts by weight, alternatively 30 parts to 60 parts by weight of the silanol-terminated polydiorganosiloxane and 80 parts to 20 parts by weight, alternatively 70 parts to 40 parts by weight of the silanol-containing silicone resin. Alternatively, the silicone PSA is produced from 30 parts to 60 parts by weight of a silanol-terminated polydiorganosiloxane having a viscosity of 0.1 Pa.s to 30000 Pa.s at 25°C and 40 parts to 70 parts of a silanol-containing silicone resin having M and Q groups as defined above, with the number ratio of M to Q in the range of 0.5:1 to 1.2:1.

To produce the silicone PSA the silanol-terminated polydiorganosiloxane and silanol-containing silicone resin are typically mixed together. The silanol groups of the polydiorganosiloxane generally undergo some condensation with the silanol groups of the silicone resin such that the polydiorganosiloxane is crosslinked with the resin. A catalyst, for example an alkaline material, can be added to promote the crosslinking reaction. Useful catalysts include ammonia, ammonium hydroxide, and ammonium carbonate.

Remaining silanol groups on the silicone PSA produced above may be at least partially reacted with an endblocking agent that introduces a triorganosilyl unit. The endblocking agent can be exemplified by disilazanes such as hexamethyldisilazane or a trialkyl alkoxy silane such as trimethyl ethoxy silane or trimethyl methoxy silane. Methods for endblocking silanol containing silicone PSAs are known in the art and are taught in U.S. Patent No. 6,337,086.

Another type of silicone adhesive that can be used herein are those that are produced from a silanol-terminated polydiorganosiloxane and an acetoxysilane. These adhesives are commercially available and sold by Dow Corning as Silastic® Medical Adhesive Silicone, Type A.

Another type of silicone PSA that can be used herein are those described in U.S. Patent Publication No. 2008-0138386 to Joffre et al. In particular, these materials are a cross-linkable composition comprising a saccharide-siloxane copolymer, a crosslinking agent and optionally a solvent.

Another type of silicone PSA that can be used herein are those described in WO Patent Publication No. WO200714599 to Dow Corning et al. In particular this silicone PSA contains 85 parts to 99.9 parts by weight of a PSA produced from (I) a silanol-terminated polydiorganosiloxane crosslinked with (II) a silanol-containing silicone resin as described above and 0.1 parts to 15 parts by weight of a silicon-containing capping agent wherein the silicon-containing capping agent is selected from the group of acrylate functional silanes, acrylate functional silazanes, acrylate functional disilazanes, acrylate functional disiloxanes, methacrylate functional silanes, methacrylate functional silazanes, methacrylate functional disilazanes, methacrylate functional disiloxanes and combinations thereof.

Another type of silicon PSA that can be used herein are those described in U.S. Patent Application Serial No. 12/203362. In particular this PSA is a reaction product of a silicon containing PSA, an ethylenically unsaturated monomer and an initiator. Typically the silicon containing PSA is PSA produced from (I) a silanol-terminated polydiorganosiloxane crosslinked with (II) a silanol-containing silicone resin as described above. The ethylenically unsaturated monomer can be any monomer having at least on carbon-carbon double bond. Typically the ethylenically unsaturated monomer is a compound selected from the group of aliphatic acrylates, aliphatic methacrylates, cycloaliphatic acrylates, cycloaliphatic methacrylates, and combinations thereof. Typically initiators include peroxides, azo compounds, redoxinitiators, and photo-initiators.

### Silicone gel adhesive

As used herein, a "silicone gel" is an elastic, jelly-like solid material formed by lightly cross-linking silicone polymers. By contrast, a pressure sensitive adhesive (PSA) has low or no resiliency.

The silicone gel used in the present invention should be chosen to have the properties desired for the end application. Important properties can include softness, friability and strength.

The gels used in the present invention are generally formed from linear or branched silicones having reactive groups thereon. Such reactive groups undergo a cross-linking reaction during curing. Examples of cross-linking reactions include the hydrosilylation reaction in which a silicone having an Si-H reactive group reacts with a silicone having an aliphatic unsaturated reactive group in the presence of a hydrosilylation catalyst. These materials are described, for example in US 5,656,279, US 5,891,076, EP0322118 and US 4,991,574. An alternative reaction is the condensation cure in which an alkoxy and/or hydroxy containing siloxanes are cured with a catalyst as described in US 4,831,070.

Typically, the gels are obtained by reacting a gel producing composition comprising (A) at least one alkenyl-substituted polydiorganosiloxane, (B) at least one organosiloxane containing silicon-bonded hydrogen atoms, and (C) at least one catalyst for the reaction of the SiH groups with the Si-alkenyl groups. These compositions cure at normal ambient temperatures, but curing can be expedited by heating to elevated temperatures, e.g., from 40°C to 140°C.

The alkenyl-substituted polydiorganosiloxanes (A) are known in the art as described, for example, in US patent number 3,983,298. Suitable alkenyl groups contain from 2 carbon to about 6 carbon atoms and are exemplified by, but not limited to, vinyl, allyl, and hexenyl. The alkenyl groups in this component may be located at terminal, pendant (non-terminal), or both terminal and pendant positions. The remaining silicon-bonded organic groups in the alkenyl-substituted polydiorganosiloxane are independently selected from the group consisting of monovalent hydrocarbon and monovalent halogenated hydrocarbon groups free of aliphatic unsaturation. These groups typically contain from 1 carbon to 20 carbon atoms, alternatively from 1 carbon to 8 carbon atoms and are exemplified by, but not limited to, alkyl such as methyl, ethyl, propyl, and butyl; aryl such as phenyl; and halogenated alkyl such as 3,3,3-trifluoropropyl. Typically at least 50 percent of the organic groups in the alkenyl-substituted polydiorganosiloxane are methyl.

The structure of the alkenyl-substituted polydiorganosiloxane is typically linear however; it may contain some branching due to the presence of trifunctional siloxane units. The viscosity of the alkenyl-substituted polydiorganosiloxane can be any desired. For example, it can be >0 mm²/s to 100,000 mm²/s, alternatively 50 mm²/s to 80,000 mm²/s, alternatively 300 mm²/s - 3,000 mm²/s.

Methods for preparing the alkenyl-substituted polydiorganosiloxanes of the present invention, such as condensation of the corresponding halosilanes or equilibration of cyclic polydiorganosiloxanes, are well known in the art.

The alkenyl-substituted polydiorganosiloxanes can be used in the gel producing composition in an amount of 10 wt. % - 90 wt. % based on the weight of the composition, alternatively 40 wt. % - 90 wt. %, alternatively 50 wt. % - 80 wt.%. The amount of alkenyl group present in the alkenyl-substituted polydiorganosiloxane is typically in the range of 0.05 wt. % - 1% wt%, alternatively 0.05 wt. % to I wt. % based on the weight of the alkenyl-substituted polydiorganosiloxane.

The organosiloxane containing silicon-bonded hydrogen atoms (B) are also known in the art as described, for example in US patent number 3,983,298. The hydrogen atoms in this component may be located at terminal, pendant (non-terminal), or both terminal and pendant positions. The remaining silicon-bonded organic groups in this component are independently selected from the group consisting of monovalent hydrocarbon and monovalent halogenated hydrocarbon groups free of aliphatic unsaturation. These groups typically contain from 1 carbon to about 20 carbon atoms, alternatively from 1 carbon to 8 carbon atoms, and are exemplified by, but not limited to, alkyl such as methyl, ethyl, propyl, and butyl; aryl such as phenyl; and halogenated alkyl such as 3,3,3-trifluoropropyl. In one embodiment of the invention, at least 50 percent of the organic groups in the organosiloxane containing silicon-bonded hydrogen atoms are methyl.

The structure of the organosiloxane containing silicon-bonded hydrogen atoms is typically linear however; it may contain some branching due to the presence of trifunctional siloxane units. The viscosity of the organosiloxane containing silicon-bonded hydrogen atoms can be any desired. For example, it can be >0 mm²/s to 100,000 mm²/s, alternatively, 5 mm²/s to 500 mm²/s.

The organosiloxanes containing silicon-bonded hydrogen atoms can be used in the gel producing composition in an amount of 1 wt. % - 30 wt. % based on the weight of the composition, alternatively 5 wt. % - 20 wt %, and alternatively 5 wt. % - 15 wt. %. In one embodiment, the amount of hydrogen group present in the organosiloxane containing silicon-bonded hydrogen atoms is between 0.05 wt. % - 1.44 wt% based on the weight of the organosiloxane containing silicon-bonded hydrogen atoms.

Methods of preparing the organosiloxane containing silicon-bonded hydrogen atoms of the present invention by co-hydrolysis of the appropriate chlorosilanes are known in the art. U.S. Patent No. 2,877,255 to Clark; Japanese Laid Open Patent Application (KOKAI) SHO 62(1987)-39660 to Mogi et al.; and U.S. Patent Nos. 5,446,185 and U.S. No. 5,493,040 to Cobb et al.

In the gel producing compositions (A) and (B) are present such that the ratio of (H as SiH):(Alkenyl as Si-Alkenyl) is generally in the range of 0.1:1 to 10:1.

The hydrosilylation catalyst (C) promotes the addition reaction of the alkenyl-substituted polydiorganosiloxane with the organosiloxane containing silicon-bonded hydrogen. The hydrosilylation catalyst can be any of the well known hydrosilylation catalysts comprising a platinum group metal, a compound containing a platinum group metal, or a microencapsulated platinum group metal or compound containing same. These platinum group metals include platinum, rhodium, ruthenium, palladium, osmium and iridium. Platinum and platinum compounds are preferred catalysts based on their high activity level in hydrosilylation reactions. One class of platinum catalysts is the complexes of chloroplatinic acid with certain vinyl-containing organosiloxane compounds disclosed by Willig in U.S. Pat. No. 3,419,593. A specific catalyst of this type is the reaction product of chloroplatinic acid and 1,3-diethenyl-1,1,3,3-tetramethyldisiloxane.

The hydrosilylation catalyst is present in an amount sufficient to cure the composition of the present invention. Typically, the concentration of the catalyst is sufficient to provide from 0.1 ppm to 500 ppm (part per million), alternatively from 1 ppm to 100 ppm, alternatively from 1 ppm to 50 ppm of a platinum group metal, based on the weight of (A) and (B).

If desired, other components can be included in the gels of the present invention including, but not limited to, fillers, pigments, low temperature cure inhibitors, additives for improving adhesion, cross-linkers (e.g., Si-H cross-linkers), chain extenders, pharmaceutical agents, cosmetic agents, natural extracts, fluids or other materials conventionally used in gels. Other optional components include silicone fluids, silicone waxes, silicone polyethers, and other polymers including, for example, hydrophilic polymers such as sodium polyacrylic acid, PVA, PVP, polyacrylic adhesive, cellulose and polysaccharide (which can make the gel more hydrophilic and more permeable to moisture). Still other optional components include rheology modifiers such as thickening agents, thixotropic agents and materials that react with the ingredients in the gel such as castor oil or maleates that can react with the hydroxyl groups of the resin. Typically, the gel contains substantially no filler (e.g., less than 5 wt.%, alternatively less than 1 wt. %, alternatively less than 0.1 wt. %). Also typically, the gel contains substantially no solvent (e.g., less than 5 wt.%, alternatively less than 1 wt. %, alternatively less than 0.1 wt. %).

Another optional ingredient is a hydroxy substituted silicone resin as described in U.S. Patent Application No. 2007-0202245. The resin is typically comprised of groups having the formula R³₃SiO_{1/2} ("M" groups) and groups having the formula SiO_{4/2} ("Q" groups) where R³ is a alkyl group having 1 carbon to 6 carbon atoms or alkylene group having 1 carbon to 6 carbon atoms, typically methyl or vinyl.

If an alkenyl group is present in the resin, typically the mole % of R groups present as alkenyl groups is < 10 mole%, alternatively 5 mole%.

The number ratio of M groups to Q groups is typically in the range of 0.6:1 to 4:1, alternatively 0.6:1 1 to 1.0:1. The silicone resin typically contains 0.1 wt% to 5 wt%, alternatively 1.0 wt% to 5 wt% silicone-bonded hydroxy groups.

The resin can be used in the gel producing composition in an amount of 2 wt% to 45 wt%, based on the weight of the gel producing composition and resin; alternatively 5 wt% to 40 wt%, alternatively 10 wt% to 35 wt%.

The silicone gel adhesive layer can be made by processes known in the art. For example, the gel may be preformed (e.g. as a sheet) by molding, calendaring, extruding, spraying, brushing, applying by hand, casting or coating on a substrate such as a liner.

Or the silicone gel layer can be made by applying the gel producing composition to a substrate by spraying, coating, bar coating, etc. Once applied to the substrate the gel producing composition is cured to produce the silicone gel adhesive on the substrate.

The silicone gel adhesives useful herein typically have a penetration of 5 mm to 300 mm, alternatively 50 mm to 300 mm as determined by the cone penetration test method based on ASTM D-217-88 using a cone category 1806-1 weighted 62.5 g. Additionally, SGAs useful herein have a coating weight in the range of 100 g/m² to 4500 g/m², alternatively 150 g/m² to 1200 g/m².

The adhesive strength of the silicone gel adhesive should be sufficient to maintain adhesion to the substrate to which it is applied (i.e. skin) yet not too strong that it would damage the substrate upon removal. The tack of the SGA, when measured with a probe tack tester, is typically 50 g to 500 g, alternatively 150 g to 350 g.

Depending on the active to be delivered, SGA may or may not act as a rate controlling layer for the diffusion of the active.

### Liners

In the construction the pressure sensitive adhesive is covered on one side by the external film layer and by the silicone gel on the other side. After the construction is prepared one side of the silicone gel adhesive is exposed. This side will be the side that contacts the surface such as human skin. If desired, this surface of the silicone gel adhesive can be covered or protected with a release liner prior to use. Suitable release liner materials are known in the art and can include a plastic, a silicone, a fluorinated silicone, a fluorine polymer, polyethylene, perfluoro based polymers, perfluoropolyether based polymer, PVC and others. Additionally the release liner could be made from a wide variety of materials such as paper coated with a suitable release coating. The surface of the release coating can be smooth, embossed or in any other desirable form.

### Actives

The construction is particularly suitable for delivery of actives that cannot be delivered with a SGA alone because they interfere with the curing of the components used to produce the SGA or to incorporate them into the SGA will destroy the structure of the gel. In particular actives that contain a group selected from amine, sulfur, nitrogen-heterocyclic, acetylenic, unsaturated hydrocarbon monoester and diester, conjugated ene-yne, hydroperoxide, nitrile and diaziridine are useful for delivery from the construction. Specific actives that can be delivered by the construction include tocopherol (vitamin E), vitamin A palmitate, lidocaine, salicylic acid and econazole nitrate, levongestrel, niacinamide, nicotine, ketoconazole.

Functional molecules that will likely inhibit the cure of platinum catalyzed Si-H to vinyl addition reactions, include nitrogen containing compounds like amines and amides, nitriles, cyanates, oximo, nitroso, hydrazo, azo compounds, and nitrogen chelates, sulfur containing compounds such as sulfides and thio compounds, phosphorus containing compounds including phosphines, phosphites and phosphates, and organic compound such as alcohols, ketones, aldehydes, carboxylic acids, esters, and organic molecules with unsaturated bonds.

Active agents useful herein include, but are not limited to, cardioactive medications, androgenic steroids, estrogens, hormones, progestational, drugs having an action on the central nervous system, nutritional agents, anti-inflammatory agents, antihistamines, respiratory agents, sympathomimetics, miotics, cholinergic agonists, antimuscarinic or muscarinic cholinergic blocking agents, mydriatics, psychicenergizers, anti-infectives, dermatological agents, humoral agents, antipspasmodics, antidepressant drugs, anti-diabetic, anorectic drugs, anti-allergenics, tranquilizers, antipsychotics, decongestants, antipyretics, antimigrane agents, drugs for treating nausea and vomiting, anti-malarials, anti-ulcerative agents, peptides, drugs for Parkinsons's disease, drugs for spasticity, drugs for acute muscle spasms, anti-estrogen, anti-hormone agents, therapeutic agents and combinations thereof.

The amount of the active or combination of actives incorporated into the PSA varies depending on many factors including, but not limited to, the particular active agent, the desired therapeutic effect, and the time span for which the construction is to provide therapy. For most active agents, the passage of the active agent through the skin is the rate-limiting step in transdermal delivery. The amount of active agent and the rate of release are typically selected so as to provide a transdermal delivery characterized by a zero order time dependency for a prolonged period of time. The minimum amount of active agent in the PSA is selected based on the active agent which passes through the skin, or other substrate, in the time span for which the construction is to provide therapy. Typically the amount of active agent in the PSA is from 0.1 wt% to 80 wt% based on the weight of the PSA and active, alternatively 0.3 wt% to 50 wt%, alternatively 1.0 wt% to 30 wt%.

The PSA or the SGA or both can contain other enhancers known to accelerate the delivery of the active agent through the skin or other substrate. Enhancers are sometimes referred to as skin-penetration enhancers, permeation enhancers, accelerants, adjuvants, and sorption promoters. Enhancers include those with diverse mechanisms of action including those which have the function of improving the solubility and diffusibility of the active agent within the PSA composition and those which improve percutaneous absorption, for example, by softening the skin, improving the skin's permeability, acting as penetration assistants or hair follicle openers or changing the state of the skin including the boundary layer. Some of these enhancers have more than one mechanism of action, but in essence they serve to enhance the delivery of the active agent to the substrate.

The enhancers may be exemplified by, but not limited to, polyhydric alcohols such as dipropylene glycol, propylene glycol and polyethylene glycol which enhance solubility of the active agent; oils such as olive oil, squalene, and lanolin; fatty ethers such as cetyl ether and oleyl ether; fatty acid esters such as isopropyl myristate which enhance diffusibility of the active agent; urea and urea derivatives such as allantoin which affect the ability of keratin to retain moisture; polar solvents such as dimethyldecylphophoxide, methyloctylsufoxide, dimethyllaurylamide, dodecylpyrrolidone, isosorbitol, dimethylacetonide, dimethylsulfoxide, decylmethylsulfoxide, and dimethylformamide which affect keratin permeability; salicylic acid which softens the keratin; amino acids which are penetration assistants; benzyl nicotinate which is a hair follicle opener; and higher molecular weight aliphatic surfactants such as lauryl sulfate salts which change the surface state of the substrate (skin) and the active agents administrated. Other enhancers include oleic and linoleic acids, ascorbic acid, panthenol, butylated hydroxytoluene, tocopherol, tocopheryl acetate, tocopheryl linoleate, propyl oleate and isopropyl palmitate.

### Construction

The construction may be manufactured in various ways. Typically the construction is produced by producing a blend of pressure sensitive adhesive and active; forming a layer of the blend on a surface of the external film layer; applying a layer of uncured silicone gel adhesive on the liner and thereafter curing the silicone gel adhesive; contacting the SGA layer with the blend layer to produce the construction.

Another method for producing the construction comprises producing a layer of SGA on a liner; forming a blend of pressure sensitive adhesive and active; forming a layer of the blend on the SGA; and applying the external film layer over the blend layer.

Another method for producing the construction comprises forming a blend of pressure sensitive adhesive and active; forming a layer of the blend on a surface of the external film layer; producing a silicone gel adhesive; applying a layer of silicone gel adhesive to the blend layer; applying a liner over the SGA.

Another method for producing the construction comprises forming a blend of pressure sensitive adhesive and active; producing a layer of the blend on a surface of a temporary liner. Apply the blend layer to the external film layer; removing the temporary liner; thereafter applying the SGA and liner to the blend layer.

Another method for producing the construction comprises forming a blend of pressure sensitive adhesive and active; forming a layer of the blend on a surface of the external film layer; applying a layer of uncured gel to the blend; curing the gel; and applying a liner over the gel. This method typically is not desirable as the active may still interfere with the curing of the gel. Thus it is typical to have the gel cured before contacting it with the PSA/active layer.

Typically the PSA and SGA are held together by their natural affinity. However, it may be desirable to apply pressure once the two surfaces are bought into contact to ensure bonding between the two materials.

In the construction the external film layer typically has a thickness of < 0.15 mm, alternatively 0.012 mm to 0.1 mm, alternatively 0.0625 mm to 0.0875 mm. The pressure sensitive adhesive layer typically has a thickness of <1.5 mm, alternatively 0.0005 mm to 1 mm, alternatively 0.0005 mm to 0.05 mm. The silicone gel adhesive layer typically has a thickness of < 5 mm, alternatively 0.05 mm to 2 mm, alternatively 0.1 mm to 1.5 mm.

### EXAMPLES

The following examples are included to demonstrate embodiments of the invention. It should be appreciated by those of skill in the art that the techniques disclosed in the examples which follow represent techniques discovered by the inventor to function well in the practice of the invention, and thus can be considered to constitute modes for its practice. However, those of skill in the art should, in light of the present disclosure, appreciate that many changes can be made in the examples which are disclosed and still obtain a like or similar result without departing from the scope of the invention. All percentages are in wt. %
**Adhesive 1** (7-4301): PSA 1 is a non-curing PSA that was produced by reacting 28 parts of dimethiconol with 48 parts of an MQ resin having a silanol content of 4500 ppm. Once the reaction was complete residual SiOH was endcapped with trimethylsilyl groups using 14 parts hexamethyldisilazane. PSA 1 is used diluted 60:40 in heptane.
**Adhesive 2** (7-4102) PSA 2 is a non-curing PSA that was produced by reacting 22 parts of dimethiconol with 59 parts of an MQ resin having a silanol content of 4500 ppm. Once the reaction was complete residual SiOH was endcapped with trimethylsilyl groups using 11 parts hexamethyldisilazane. PSA 2 is used diluted 60:40 in ethylacetate.
**Adhesive 3** (7-4501) PSA 3 is a non-curing PSA that was produced by reacting 29 parts of dimethiconol with 62 parts of an MQ resin having a silanol content of 4500 ppm. PSA 3 is used diluted 60:40 in heptane.
**Adhesive 4** (7-4202) PSA 4 is a non-curing PSA that was produced by reacting 25 parts of dimethiconol with 53 parts of an MQ resin having a silanol content of 4500 ppm. Once the reaction was complete residual SiOH was endcapped with trimethylsilyl groups using 12 parts hexamethyldisilazane. PSA 4 is used diluted 60:40 in ethylacetate.
**Adhesive 5** (7-4302) PSA 5 is a non-curing PSA that was produced by reacting 28 parts of dimethiconol with 48 parts of an MQ resin having a silanol content of 4500 ppm. Once the reaction was complete residual SiOH was endcapped with trimethylsilyl groups using 14 parts hexamethyldisilazane. PSA 5 is used diluted 60:40 in ethylacetate.
**Adhesive 6** (7-4502) PSA 6 is a non-curing PSA that was produced by reacting 29 parts of dimethiconol with 62 parts of an MQ resin having a silanol content of 4500 ppm. PSA 6 is used diluted 60:40 in ethylacetate.
**Gel 1** (7-9800) Gel 1 is a two part curing adhesive including a Part A produced by blending 100 parts of vinyl-terminated polydimethylsiloxane with 0.25 part of platinum complex and a Part B produced by blending 85 parts of vinyl-terminated polydimethylsiloxane with 15 parts of hydrogen-functional dimethylsiloxane.
**Gel 2** (Dow Corning Experimental Gel prepared as per PCT filing US20070202245 A1) Gel 2 is a two part curing adhesive including a part A produced by blending 73 parts of vinyl-terminated polydimethylsiloxane with 23 parts Dimethylvinylated and Trimethylated Silica, 5 parts Silicic Acid, Sodium salt and 0.30 part of platinum complex and a part B produced by blending 50 parts of vinyl-terminated polydimethylsiloxane with 32 parts Silicic Acid, Sodium salt and 17 parts of hydrogen-functional dimethylsiloxane.

### Example 1

2.25 g of Nicotine was dispersed in 1.0 g heptane and then the mixture was added to 1.25g Adhesive 1 and mixed to homogeneity using a mixer. The drug/adhesive mixture was then cast onto Ethyl Vinyl Acetate backing material. Stainless steel shims of 0.102 mm and 0.152 mm were used to acquire two desired coating thicknesses. Samples were allowed to devolatize at ambient conditions for at least 3 hours to remove the heptane. Laminates with approximate dried adhesive thickness of 0.06 mm and 0.09 mm were obtained.

In a separate operation, Gel 1 was cast onto a low density polyethylene (LDPE) film using 0.152 mm stainless steel shims. These were placed in an oven at 80°C for approximately fifteen minutes until they were fully cured.

The two adhesive films were then squeezed together using a hydraulic press. The LDPE film was then removed from the surface of Gel I film to expose a skin facing surface of a transdermal delivery device.

### Example 2

40 g Adhesive 2 was mixed with 2.67 g 100 cSt polydimethylsiloxane and mixed to homogeneity on a mixing wheel. 2.96 g Lidocaine was dissolved in 5 mL ethyl alcohol, mixed and added to adhesive 2 using a mixer. The drug/adhesive mixture was then cast onto a fluorosilicone release liner. Stainless steel shims 0.178 mm were used to determine the thickness of the coating. The samples were allowed to devolatize at ambient conditions for at least 3 hours to remove the heptane. The samples were transferred from the fluorosilicone release liner to a heat sealable polyester backing material. Laminates with approximate dried adhesive thickness 0.1 mm were obtained.

In a separate operation, Gel 1 was cast onto a polypropylene (PP) film at a coated thickness of approximately 0.2 mm.

The two adhesive films were then squeezed together using a hydraulic press. The PP film was then removed from the surface of Gel 1 to expose a skin facing surface of a transdermal delivery device.

### Examples 3 through 7

**Table 1. Lidocaine/PSA**

| Component | Example 3 | Example 4 | Example 5 | Example 6 | Example 7 |
|---|---|---|---|---|---|
| Adhesive 3 | 25g | 25g | --- | --- | --- |
| Adhesive 2 | --- | --- | 40g | --- | --- |
| Adhesive 4 | --- | --- | --- | 40g | --- |
| Adhesive 5 | --- | --- | --- | --- | 40g |
| 100 cSt PDMS | --- | --- | 2.67g | --- | --- |
| Lidocaine | 0.78g | 1.656g | 2.96g | 2.67g | 2.96g |

The adhesives referenced in Table 1 were mixed with lidocaine and cast onto a fluorosilicone release liner. Stainless steel shims 0.152 mm were used to determine the thickness of the coating. The samples were allowed to devolatize at ambient conditions for at least 3 hours to remove the heptane. The samples were transferred from the fluorosilicone release liner to a polyester backing material. Laminates with approximate dried adhesive thickness 0.09 mm were obtained.

In a separate operation, Gel 1 was cast onto a polypropylene (PP) film at a coated thickness of approximately 0.2 mm.

The two adhesive films were then squeezed together using a hydraulic press. The PP film was then removed from the surface of the Gel 1 to expose a skin facing surface of a transdermal delivery device.

### Examples 8 through 13

**Table 2. Amine compatible silicone PSA/Econazole.**

| Component | Example 8 | Example 9 | Example 10 | Example 11 | Example 12 | Example 13 |
|---|---|---|---|---|---|---|
| Adhesive 6 | 9.7g | 9.5g | 9.0g | --- | --- | --- |
| Adhesive 4 | --- | --- | --- | 10g | 10g | 10g |
| Econazole nitrate | 0.182g | 0.303g | 0.606g | 0.185g | 0.314g | 0.663g |

Adhesive 6 and Adhesive 4 were mixed with econazole nitrate and cast onto a polyester backing material. Stainless steel shims 0.152mm were used to determine the thickness of the coating. The samples were allowed to devolatize at ambient conditions for at least 3 hours to remove the heptane. Laminates with approximate dried adhesive thickness 0.09 mm were obtained.

In a separate operation, Gel 1 was cast onto a Low Density Polyethylene (LDPE) film at a coated thickness of approximately 0.2 mm. These were placed in an oven at 80°C for approximately fifteen minutes until they were fully cured.

The two adhesive films were then squeezed together using a hydraulic press. The LDPE film was then removed from the surface of the Gel 1 film to expose a skin facing - surface of a transdermal delivery device.

### Examples 14 through 16

**Table 5: Salicylic acid/silicone PSA**

| Component | Example 14 | Example 15 | Example 16 |
|---|---|---|---|
| Adhesive 6 | 10.0 | 10.0 | 10.0 |
| Salicylic Acid | 0.314g | 0.663g | 0.314g |
| Gel | Gel 1 | Gel 1 | Gel 2 |

Adhesive 6 was mixed with salicylic acid and cast onto a polyester backing material. Stainless steel shims 0.152 mm were used to determine the thickness of the coating. Samples were allowed to devolatize at ambient conditions for at least 3 hours to remove the heptane. Laminates with approximate dried adhesive thickness 0.09 mm were obtained.

In a separate operation, Gels 1 and 2 were separately cast onto a Low Density Polyethylene (LDPE) film at a coated thickness of approximately 0.2 mm. These were placed in an oven at 80°C for approximately fifteen minutes until they were fully cured.

The two adhesive films were then squeezed together using a hydraulic press. The LDPE film was then removed from the surface of the cured gel film to expose a skin facing surface of a transdermal delivery device.

### Examples 17-19

**Table 6: ketoprofen/amine compatible silicone PSA .**

| Component | Example 17 | Example 18 | Example 19 |
|---|---|---|---|
| Adhesive 2 | 10.0 | 10.0 | 10.0 |
| Ketoprofen | 0.314g | 0.663g | 1.990 |

Adhesive 2 was mixed with ketoprofen and cast onto a polyester backing material. Stainless steel shims 0.152 mm were used to determine the thickness of the coating. Samples were allowed to devolatize at ambient conditions for at least 3 hours to remove the heptane. Laminates with approximate dried adhesive thickness 0.09 mm were obtained.

In a separate operation, Gel 1 cast onto a Low Density Polyethylene (LDPE) film at a coated thickness of approximately 0.2 mm. These were placed in an oven at 80°C for approximately fifteen minutes until they were fully cured.

The two adhesive films were then squeezed together using a hydraulic press. The LDPE film was then removed from the surface of the Gel 1 film to expose a skin facing surface of a transdermal delivery device.

### Example 20

Drug release was determined by placing the constructions prepared above onto a Franz diffusion cells containing a receptor fluid (RF). Full replacement of the receptor fluid was performed at the specified time intervals from 1 hour to 24 hours. Estimation of the amount of active in the receptor fluid at each interval was performed using ultraviolet (UV) spectrophotometry with the receptor fluid as the background medium. Results of the release are given in Table 5.

**Table 5.**

| Example | Cumulative Amount Released (µg) | Percentage of Drug Released | Release Rate (µg/cm²) | Receptor Fluid |
|---|---|---|---|---|
| 2 | 1441.63 | 9.9 | 293.6 | 1 |
| 6 | 2343.5 | 15.7 | 477.3 | 1 |
| 7 | 2253.1 | 14.3 | 458.9 | 1 |
| 11 | 206.8 | 16.2 | 42.1 | 2 |
| 12 | 209.8 | 12.2 | 42.7 | 2 |
| 13 | 204.2 | 5.9 | 41.6 | 2 |
| 15 | 5419.9 | 50.8 | 1104.1 | 3 |
| 16 | 2278.6 | 59.4 | 464.2 | 3 |
| 17 | 2417.2 | 36.1 | 492.4 | 3 |

| | | | | |
|---|---|---|---|---|
| RF 1: 5% polyethylene glycol 400, 25% 200 proof ethanol, 70% deionized water RF 2: 40% PEG 400 in deionized water RF 3: 0.9% saline solution | | | | |

## Claims

1. A construction comprising an external film layer, an adhesive layer applied on the external film layer; and a silicone gel adhesive that is applied on the adhesive layer wherein the adhesive layer comprises a non-curing pressure sensitive adhesive that has been blended with an active ingredient wherein the non-curing pressure sensitive adhesive is a silicone.

2. The construction as claimed in claim 1 wherein a liner is applied on the silicone gel adhesive.

3. The construction as claimed in claim 1 or 2 wherein the external film layer is non-occlusive.

4. The construction as claimed in claim 1 or 2 wherein the silicone pressure sensitive adhesive is comprised of the product of (I) a silanol-terminated polydiorganosiloxane crosslinked with (II) a silanol-containing silicone resin.

5. The construction as claimed in claim 1 or 2 wherein the silicone gel adhesive is a reaction product of (A) 10 wt% - 90 wt% of at least one alkenyl-substituted polydiorganosiloxane, (B) 1 wt% - 30 wt% of at least one organosiloxane containing silicon-bonded hydrogen atoms, and (C) at least one catalyst for the reaction of the SiH groups with the Si-alkenyl groups.

6. The construction as claimed in claim 5 wherein there is also present (D) 3 wt% to 45 wt% of a hydroxy substituted silicone resin.

7. The construction as claimed in claim 1, 2 or 5 wherein the active is selected from tocopherol (vitamin E), vitamin A palmitate, lidocaine, salicylic acid, econazole nitrate, levongestrel, niacinamide, nicotine, and ketoconazole.

8. The construction as claimed in claim 1, 2 or 5 wherein the active contains a functional molecule selected from amines and amides, nitriles, cyanates, oximo, nitroso, hydrazo, azo compounds, nitrogen chelates, sulfides and thio compounds, phosphines, phosphites and phosphates, alcohols, ketones, aldehydes, carboxylic acids, esters, and organic molecules with unsaturated bonds.

9. The construction as claimed in 1, 7 or 8 wherein there is a 1 to 80 wt% active based on the weight of the pressure sensitive adhesive and active.

10. The construction as claimed in claim 1 wherein the external film layer has a thickness of < 0.15 mm.

11. The construction as claimed in claim 1 or 10 wherein the pressure sensitive adhesive layer has a thickness of <1.5 mm.

12. The construction as claimed in claim 1, 10 or 11 wherein the silicone gel adhesive layer has a thickness of < 5 mm.

## Patentansprüche

1. Eine Konstruktion, die eine externe Filmschicht, eine auf der externen Filmschicht angebrachte haftende Schicht und ein Haftmittel aus Silicon-Gel, das auf der haftenden Schicht angebracht ist, beinhaltet, wobei die haftende Schicht ein nicht aushärtendes druckempfindliches Haftmittel beinhaltet, das mit einem Wirkstoff vermischt worden ist, wobei das nicht aushärtende druckempfindliche Haftmittel ein Silicon ist.

2. Konstruktion gemäß Anspruch 1, wobei eine Einlage auf dem Haftmittel aus Silicon-Gel angebracht ist.

3. Konstruktion gemäß Anspruch 1 oder 2, wobei die externe Filmschicht nicht okklusiv ist.

4. Konstruktion gemäß Anspruch 1 oder 2, wobei das druckempfindliche Haftmittel aus Silicon das Produkt aus (I) einem Silanol-terminierten Polydiorganosiloxan, vernetzt mit (II) einem Silanol-enthaltenden Siliconharz, beinhaltet.

5. Konstruktion gemäß Anspruch 1 oder 2, wobei das Haftmittel aus Silicon-Gel ein Reaktionsprodukt aus (A) 10 Gew.-%-90 Gew.-% von mindestens einem Alkenylsubstituierten Polydiorganosiloxan, (B) 1 Gew.-%-30 Gew.-% von mindestens einem Silicon-gebundene Wasserstoffatome enthaltenden Organosiloxan und (C) mindestens einem Katalysator für die Reaktion der SiH-Gruppen mit den Si-Alkenyl-Gruppen ist.

6. Konstruktion gemäß Anspruch 5, wobei (D) 3 Gew.-% bis 45 Gew.-% eines Hydroxysubstituierten Siliconharzes ebenfalls vorhanden ist.

7. Konstruktion gemäß Anspruch 1, 2 oder 5, wobei der Wirkstoff aus Tocopherol (Vitamin E), Vitamin-A-Palmitat, Lidocain, Salicylsäure, Econazolnitrat, Levonorgestrel, Niacinamid, Nicotin und Ketoconazol ausgewählt ist.

8. Konstruktion gemäß Anspruch 1, 2 oder 5, wobei der Wirkstoff ein funktionelles Molekül, ausgewählt aus Aminen und Amiden, Nitrilen, Cyanaten, Oximo, Nitroso, Hydrazo, Azoverbindungen, Stickstoffchelaten, Sulfiden und Thioverbindungen, Phosphinen, Phosphiten und Phosphaten, Alkoholen, Ketonen, Aldehyden, Carbonsäuren, Estern und organischen Molekülen mit ungesättigten Bindungen, enthält.

9. Konstruktion gemäß 1, 7 oder 8, wobei es einen 1 bis 80 Gew.-% Wirkstoff basierend auf dem Gewicht des druckempfindlichen Haftmittels und Wirkstoffs gibt.

10. Konstruktion gemäß Anspruch 1, wobei die externe Filmschicht eine Dicke von < 0,15 mm aufweist.

11. Konstruktion gemäß Anspruch 1 oder 10, wobei die druckempfindliche haftende Schicht eine Dicke von < 1,5 mm aufweist.

12. Konstruktion gemäß Anspruch 1, 10 oder 11, wobei die haftende Schicht aus SiliconGel eine Dicke von < 5 mm aufweist.

## Revendications

1. Une construction comprenant une couche pelliculaire externe, une couche d'adhésif appliquée sur la couche pelliculaire externe ; et un adhésif en gel de silicone qui est appliqué sur la couche d'adhésif, la couche d'adhésif comprenant un adhésif sensible à la pression non durcissant qui a été mélangé de façon homogène avec un ingrédient actif, l'adhésif sensible à la pression non durcissant étant un silicone.

2. La construction telle que revendiquée dans la revendication 1 dans laquelle une doublure est appliquée sur l'adhésif en gel de silicone.

3. La construction telle que revendiquée dans la revendication 1 ou la revendication 2 dans laquelle la couche pelliculaire externe est non occlusive.

4. La construction telle que revendiquée dans la revendication 1 ou la revendication 2 dans laquelle l'adhésif sensible à la pression en silicone est composé du produit de (I) un polydiorganosiloxane à terminaison silanol réticulé avec (II) une résine de silicone contenant du silanol.

5. La construction telle que revendiquée dans la revendication 1 ou la revendication 2 dans laquelle l'adhésif en gel de silicone est un produit de réaction de (A) 10 % en poids à 90 % en poids d'au moins un polydiorganosiloxane à substitution alcényle, (B) 1 % en poids à 30 % en poids d'au moins un organosiloxane contenant des atomes d'hydrogène liés au silicium, et (C) d'au moins un catalyseur pour la réaction des groupes SiH avec les groupes Si-alcényle.

6. La construction telle que revendiquée dans la revendication 5 dans laquelle sont aussi présents (D) 3 % en poids à 45 % en poids d'une résine de silicone à substitution hydroxy.

7. La construction telle que revendiquée dans la revendication 1, la revendication 2 ou la revendication 5 dans laquelle la substance active est sélectionnée parmi le tocophérol (vitamine E), le palmitate de vitamine A, la lidocaïne, l'acide salicylique, le nitrate d'éconazole, le lévongestrel, le niacinamide, la nicotine, et le cétonazole.

8. La construction telle que revendiquée dans la revendication 1, la revendication 2 ou la revendication 5 dans laquelle la substance active contient une molécule fonctionnelle sélectionnée parmi des amines et des amides, des nitriles, des cyanates, des composés oximo, nitroso, hydrazo, azo, des chélates d'azote, des sulfures et des composés thio, des phosphines, des phosphites et des phosphates, des alcools, des cétones, des aldéhydes, des acides carboxyliques, des esters, et des molécules organiques avec des liaisons insaturées.

9. La construction telle que revendiquée en 1, 7 ou 8 dans laquelle il se trouve de 1 à 80 % en poids de substance active rapporté au poids de l'adhésif sensible à la pression et à la substance active.

10. La construction telle que revendiquée dans la revendication 1 dans laquelle la couche pelliculaire externe a une épaisseur < 0,15 mm.

11. La construction telle que revendiquée dans la revendication 1 ou la revendication 10 dans laquelle la couche d'adhésif sensible à la pression a une épaisseur < 1,5 mm.

12. La construction telle que revendiquée dans la revendication 1, la revendication 10 ou la revendication 11 dans laquelle la couche d'adhésif en gel de silicone a une épaisseur < 5 mm.
